(19) Europäisches Patentamt
European Patent Office
Office européen des brevets

(11) Veröffentlichungsnummer : **0 130 509**
**B1**

(12) # EUROPÄISCHE PATENTSCHRIFT

(45) Veröffentlichungstag der Patentschrift : **10.09.86**

(51) Int. Cl.[4] : **C 07 C172/00**, C 07 D335/12, C 07 D345/00

(21) Anmeldenummer : **84107214.3**

(22) Anmeldetag : **23.06.84**

(54) **Verfahren zur Herstellung von Prävitamin D und neue Photosensibilisatoren.**

(30) Priorität : **01.07.83 CH 3637/83**

(43) Veröffentlichungstag der Anmeldung : **09.01.85 Patentblatt 85/02**

(45) Bekanntmachung des Hinweises auf die Patenterteilung : **10.09.86 Patentblatt 86/37**

(84) Benannte Vertragsstaaten : **AT BE CH DE FR GB IT LI NL**

(56) Entgegenhaltungen :
**WO-A-82 /038 64**
**CH-A- 363 342**
**DE-A- 3 048 698**
**GB-A- 1 592 170**
**THE JOURNAL OF ORGANIC CHEMISTRY, vol. 44, 1979 Y. TAMURA et al. "Stereochemistry and Base-Catalyzed Rearrangement of 9-Phenylthioxanthene-N(p-toluene-sulfonyl)sulfilimine" pages 3296-3299**
**TETRAHEDRON LETTERS, vol. 24, 1983, T. KATAOKA et al. "Stable Setenoxanthenium Ylides: Synthesis and new Reductive Cyclization of Selenoxanthen-10-io(alkoxalyl alkoxycarbonyl)methanides and their Related Compounds" pages 75-78**

(73) Patentinhaber : **F. HOFFMANN-LA ROCHE & CO. Aktiengesellschaft**

**CH-4002 Basel (CH)**

(72) Erfinder : **Hansen, Hans-Jürgen, Prof.**
**Bettingerstrasse 67**
**CH-4125 Riehen (CH)**
Erfinder : **Pfoertner, Karlheinz, Dr.**
**Gellertstrasse 24**
**CH-4052 Basel (CH)**

(74) Vertreter : **Grossner, Lutz, Dr. et al**
**Grenzacher Strasse 124 Postfach 601**
**CH-4002 Basel (CH)**

**EP 0 130 509 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Vitamin D wird im technischen Massstab durch Bestrahlung des Provitamins 7-Dehydrocholesterin bzw. Ergosterin und Isomerisierung des bei der Bestrahlung gebildeten Prävitamins D hergestellt. Bekanntlich werden bei der Bestrahlung des Provitamins neben dem Prävitamin andere Produkte, insbesondere Lumisterin und Tachysterin gebildet, wodurch die Ausbeute an Prävitamin vermindert wird und die Notwendigkeit einer Abtrennung des Tachysterins besteht.

Es ist versucht worden, die Ausbeute dadurch zu verbessern, dass man die Bestrahlung in zwei Bestrahlungsstufen durchführte und dem primär erhaltenen Bestrahlungsprodukt Fuorenon zusetzte und erneut bestrahlte, wodurch Tachysterin z. T. in Prävitamin D umgewandelt wurde. Dieses Verfahren besitzt indessen den Nachteil, dass sich das Fluorenon von dem gewünschten Prävitamin D nur schlecht abtrennen lässt. CH-A-363 342 beschreibt die durch Eosin sensibilisierte photochemische Bildung von Tachysterin aus Prävitamin D.

Es wurde nun gefunden, dass man Prävitamin D, das im wesentlichen frei von Tachysterin ist, erhält, wenn man ein Gemisch von Prävitamin D und Tachysterin in Gegenwart einer Verbindung der Formel

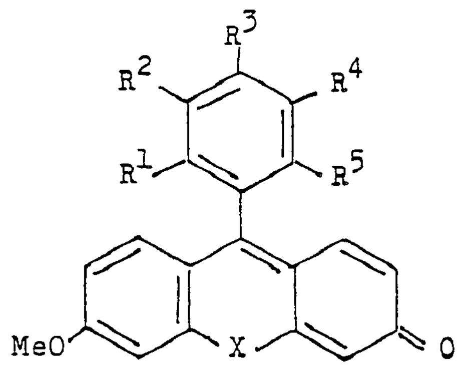

(I)

worin $R^1$-$R^4$ Wasserstoff oder Chlor, $R^5$ eine Gruppe —COOMe oder —$SO_3$Me, Me ein Alkalimetallkation und X Sauerstoff, Schwefel oder Selen bedeuten, mit Licht einer Wellenlänge von 400 nm bis 600 nm bestrahlt.

Es wurde weiter gefunden, dass Verbindungen der Formel I Photosensibilisatoren für die Umwandlung von Tachysterin in Prävitamin D darstellen. In Gegenwart von Verbindungen der Formel I wird Tachysterin in wesentlich höherem Mass photosensibilisiert in Prävitamin D überführt als bei den bekannten Verfahren mit Fluorenon. Weiterhin kann das neue Verfahren mit sichtbarem Licht durchgeführt werden, so dass UV-durchlässige, teure Quarzglasapparaturen entbehrlich sind. Ein weiterer Vorteil des neuen Verfahrens besteht darin, dass die Photosensibilisatoren, d. h. die Verbindungen der Formel I wasserlöslich sind und daher aus mit Wasser nicht mischbaren Lösungsmitteln mit Wasser extrahiert werden können.

Die Bestrahlung kann mit jeder zwischen 400 nm und 600 nm emittierenden Lichtquelle erfolgen, z. B. mit einer geeigneten Hg-Dampflampe oder mit Sonnenlicht. Vorzugsweise verwendet man eine Hg-Hochdrucklampe, wie Hanau TQ 2 020.

Zweckmässig wird das Bestrahlungsgut während der Bestrahlung gekühlt, z. B. auf eine Temperatur von etwa 15-25 °C, vorzugsweise 16-17 °C.

Weiterhin ist es zweckmässig, Lumisterin erzeugendes UV-Licht zwischen 300 und 340 nm zu eliminieren. Dies kann dadurch geschehen, dass man zwischen Lichtquelle und Bestrahlungsgut ein in diesem Bereich absorbierendes Filter, z. B. ein Glasfilter oder, vorzugsweise, eine Filterlösung anordnet. Geeignete Filterlösungen sind wässrige Lösungen von Eisensulfat und Kupfersulfat.

Das im erfindungsgemässen Verfahren eingesetzte Prävitamin D umfasst Prächolecalciferol und Präergocalciferol.

In einer bevorzugten Ausführungsform des erfindungsgemässen Verfahrens verwendet man als Ausgangsmaterial das Reaktionsprodukt, das bei der Bestrahlung von Provitamin D, d. h., 7-Dehydrocholesterin oder Ergosterin mit UV-Licht zwischen 240 und 300 nm entsteht und das neben nicht umgesetztem Provitamin D und dem Prävitamin D grössere Mengen Tachysterin enthält.

Mittels des erfindungsgemässen Verfahrens könen ausgehend von Gemischen ; die Prävitamin D und grössere Mengen, z. B. mehr als 3 %, insbesondere 15-18 % Tachysterin enthalten, Gemische von Prävitamin D und weniger als 3 % Tachysterin erhalten werden.

Das erfindungsgemässe Verfahren kann in für die Herstellung von Vitamin D durch Bestrahlung an sich bekannter Weise durchgeführt werden.

Als Lösungsmittel kommen alle Lösungsmittel in Betracht, die bisher als Lösungsmittel für die photochemische Herstellung von Vitamin D aus Dehydrocholesterin verwendet wurden. Beispiele solcher

Lösungsmittel sind Aether, wie Tetrahydrofuran, Dioxan und tert.-Butylmethyläther. Bevorzugte Lösungsmittel sind mit Wasser nicht mischbare Lösungsmittel, insbesondere tert.-Butyl-methyläther.

Um eine ausreichende Solubilisierung des Photosensibilisators im Reaktionsgemisch sicherzustellen, wird der Photosensibilisator, d. h. die Verbindung der Formel I, zweckmässig in einem organischen protischen Lösungsmittel, das mit den oben erwähnten Lösungsmitteln mischbar ist, z. B. in Methanol, gelöst und so dem Raktionsgemisch zugesetzt. Die Konzentration des bei der Reaktion anwesenden Photosensibilisators ist nicht kritisch, sie beeinflusst jedoch zusammen mit der Schichtdicke der der Bestrahlung ausgesetzten Lösung die Bestrahlungsdauer. In den weiter unten angegebenen Beispielen ist die Konzentration an Photosensibilisator so gewählt, dass mit einer Schichtdikce von 1 cm optimale Ausbeuten erzielt werden.

Die Verbindungen der Formel I, in denen X Schwefel oder Selen ist und $R^5$ eine Gruppe $—SO_3Me$ ist, wenn X Schwefel darstellt, sind neu und ebenfalls Gegenstand der Erfindung. Sie können dadurch hergestellt werden, dass man m,m'-Selenodiphenol mit einer Verbindung der Formel

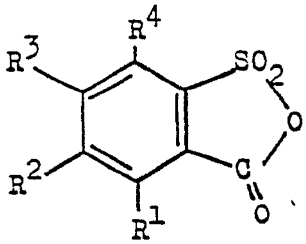

(II)

oder

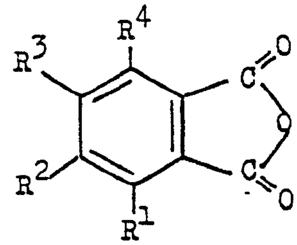

(III)

worin $R^1$-$R^4$ die oben angegebene Bedeutung haben, umsetzt oder m,m'-Thiodiphenol mit einer Verbindung der Formel II umsetzt.

Die Umsetzung kann dadurch vorgenommen werden, dass man die Reaktionspartner zusammen erhitzt, zweckmässig in Gegenwart einer Säure, wie p-Toluolsulfonsäure oder Trifluormethansulfonsäure. Die Reaktionstemperatur ist nicht kritisch, zweckmässig erhitzt man das Reaktionsgemisch bis es schmilzt, beispielsweise auf 80-140 °C. Aus der abgekühlten Schmelze kann die Verbindung der Formel I mit Wasser bei alkalischem pH extrahiert und aus der Lösung, ggf. nach Reinigung, z. B. mittels Aktivkohle, durch Ansäuern ausgefällt werden.

Bevorzugte Verbindungen der Formel I sind die, in denen $R^1$-$R^4$ Wasserstoff, $R^5$ eine Gruppe —COONa oder $—SO_3Na$ und X Schwefel oder Selen ist.

Die nachfolgenden Beispiele erläutern die Erfindung weiter.

## Beispiel 1

A. Eine Lösung von 225 g 7-Dehydrocholesterin in 4,5 l tert.-Butylmethyläther wurde unter Inertgas (Argon oder Stickstoff) in einem um die stabförmige Lichtquelle zylindrisch angeordneten Ringmantelgefäss aus Quarzglas 60 bis 70 Minuten mit einer Hochleistungs-Quecksilberniederdrucklampe von 1,5 kW belichtet. Dabei liess man die Reaktionslösung mit Hilfe einer Pumpe durch den Photoreaktor und durch ein ausserhalb befindliches Vorratsgefässe, welches gleichzeitig als Wärmetauscher ausgebildet war, zirkulieren, wobei die Temperatur bei 21-22 °C gehalten wurde. Man erhielt ein Reaktionsgemisch mit 61,5 % 7-Dehydrocholesterin ; 20,4 % Prävitamin $D_3$ ; 0,2 % Vitamin $D_3$ ; 16,8 % Tachysterin und 1,1 % Lumisterin.

B. Zu der gemäss A. erhaltenen Lösung wurden 0,6 g Fluorescein-dinatrium, gelöst in 0,6 l Methanol gegeben. Die Lösung wurde in eine mit einer 2 kW Quecksilberhochdrucklampe ausgestatteten Glasapparatur gepumpt. Im Zentrum der Apparatur befand sich die Lichtquelle, um die ein Ringmantelgefäss mit zwei Kammern konzentrisch geordnet war. Das Bestrahlungsgut liess man mit Hilfe einer Pumpe durch die äussere Kammer und durch ein ausserhalb des Reaktors befindliches Vorratsgefäss zirkulieren, welches gleichzeitig als Wärmeaustauscher ausgebildet war und eine Temperatur von 16-17 °C hatte. Durch die innere, zwischen Lichtquelle und Bestrahlungsgut befindliche Kammer wurde eine ebenfalls extern gekühlte Filterlösung folgender Zusammensetzung gepumpt :

32,90 g Kupfer-II-sulfat-5-hydrat
1,05 g Eisen-III-sulfat-5-hydrat
7,70 ml Schwefelsäure 95-98 %
993,40 ml Wasser, entionisiert,

welche in einer Schichtdicke von 1 cm alles Licht unterhalb 350 nm absorbiert. Der während der Bestrahlung zerstörte Sensibilisator wurde kontinuierlich ergänzt, wobei im Verlauf von 5 Stunden 2,1 g

Fluoresceindinatrium, gelöst in 115 ml Methanol zugesetzt wurden. Nach 5 Stunden Belichtung war der photostationäre Zustand erreicht. Das Reaktionsgemisch hatte folgende Zusammensetztung :

61,5 % 7-Dehydrocholesterin ; 34 % Prävitamin $D_3$ ; 1 % Vitamin $D_3$ ; 2,4 % Tachysterin ; und 1,1 % Lumisterin

## Beispiel 2

A. In Analogie zu Beispiel 1A wurde ein Reaktionsgemisch folgender Zusammensetzung erhalten :

62,9 % 7-Dehydrocholesterin ; 19,6 % Prävitamin $D_3$ ; 0,5 % Vitamin $D_3$ ; 16 % Tachysterin und 1,0 % Lumisterin in 4,5 1 tert.-Butylmethyläther.

B. Das in A. erhaltene Reaktionsgemisch wurde nach Zusatz von 0,66 g o-(6-Hydroxy-3-oxo-3H-thioxanthen-9-yl)-benzoesäure-dinatrium · $H_2O$, gelöst in 0,6 l Methanol, wie in Beispiel 1B bestrahlt. Eine Nachdosierung des Sensibilisators war, wie auch bei den in den nachfolgenden Beispielen verwendeten Sensibilisatoren, nicht erforderlich. Der photostationäre Zustand wurde nach 30 Minuten Belichtung erreicht und man erhielt ein Reaktionsgemisch der Zusammensetzung 62,9 % 7-Dehydrocholesterin ; 33,1 % Prävitamin $D_3$ ; 1,2 % Vitamin $D_3$ ; 1,8 % Tachysterin ; und 1,0 % Lumisterin.

## Beispiel 3

A. In Analogie zu Beispiel 1A wurde ein Reaktionsgemisch folgender Zusammensetzung erhalten :

62,0 % 7-Dehydrocholesterin ; 19,7 % Prävitamin $D_3$ ; 0,4 % Vitamin $D_3$ ; 16,9 % Tachysterin ; und 1,0 % Lumisterin in 4,5 l tert.-Butylmethyläther.

B. Das in A. erhaltene Reaktionsgemisch wurde nach Zusatz von 0,74 g o-(6-Hydroxy-3-oxo-3H-thioxanthen-9-yl)-benzolsulfonsäure-dinatrium · 2 $H_2O$, gelöst in 1,05 l Methanol, bis zum Erreichen des photostationären Zustands (21 Minuten) wie in Beispiel 1B bestrahlt. Man erhielt ein Reaktionsgemisch der Zusammensetzung 62,0 % 7-Dehydrocholesterin ; 34,2 % Prävitamin $D_3$ ; 1,4 % Vitamin $D_3$ ; 1,4 % Tachysterin ; und 1,0 % Lumisterin.

## Beispiel 4

A. In Analogie zu Beispiel 1A wurde ein Reaktionsgemisch folgender Zusammensetzung erhalten :

62,0 % 7-Dehydrocholesterin ; 19,6 % Prävitamin $D_3$ ; 0,5 % Vitamin $D_3$ ; 16,8 % Tachysterin ; und 1,1 % Lumisterin in 4,5 l tert.-Butylmethyläther.

B. Das in A. erhaltene Reaktionsgemisch wurde nach Zusatz von 0,73 g o-(6-Hydroxy-3-oxo-3H-selenoxanthen-9-yl)-benzoesäure. 1 Aceton, gelöst in 0,3 l Methanol, und 0,18 g Natriummethylat, gelöst in 0,3 l Methanol, bis zum Erreichen des photostationären Zustands (15 Min.) wie in Beispiel 1B bestrahlt. Man erhielt ein Reaktionsgemisch der Zusammensetzung 62,0 % 7-Dehydrocholesterin ; 33,7 % Prävitamin $D_3$ ; 1,4 % Vitamin D ; 1,8 % Tachysterin ; und 1,1 % Lumisterin.

## Beispiel 5

A. In Analogie zu Beispiel 1A wurde ein Reaktionsgemisch folgender Zusammensetzung erhalten :

62,0 % 7-Dehydrocholesterin ; 19,7 % Prävitamin $D_3$ ; 0,3 % Vitamin $D_3$ ; 16,9 % Tachysterin ; und 1,1 % Lumisterin in 4,5 l tert.-Butylmethyläther.

B. Das in A. erhaltene Reaktionsgemisch wurde nach Zusatz von 0,72 g o-(6-Hydroxy-3-oxo-3H-selenoxanthen-9-yl)-benzolsulfonsäure · $H_2O$, gelöst in 0,55 l Methanol, und 0,18 g Natriummethylat, gelöst in 0,5 l Methanol, bis zum Erreichen des photostationären Zustands (13 Minuten) wie in Beispiel 1B bestrahlt. Man erhielt ein Reaktionsgemisch der Zusammensetzung 62,0 % 7-Dehydrocholesterin ; 33,5 % Prävitamin $D_3$ ; 1,9 % Vitamin $D_3$ ; 1,5 % Tachysterin ; und 1,1 % Lumisterin.

## Beispiel 6

15,7 g p-Toluolsulfonsäure-monohydrat wurden unter vermindertem Druck auf 140 °C zur Entfernung des Kristallwassers erwärmt. 18 g m,m'-Thiodiphenol und 18,2 g 2-Sulfobenzoesäureanhydrid wurden miteinander verrieben und unter Argon und Rühren zu der heissen, wasserfreien p-Toluolsulfonsäure gegeben. Die Schmelze wurde unter Rühren 2 Stunden bei 140 °C (Innentemperatur) und 0,1 Torr gehalten. Nach Abkühlen wurden 16 g Natriumhydroxid in 0,3 l Wasser gelöst, zugegeben. Nach 1 Stunde Rühren bei 50 °C wurde eine Lösung von 0,09 l Wasser und 27 g Schwefelsäure (95-97 %) tropfenweise unter Rühren zugesetzt. Zur Vervollständigung der Fällung wird im Eisbad auf ca. 5 °C gekühlt und der sehr feinkristalline Niederschlag abgenutscht. Der Filterkuchen wird auf der Nutsche zweimal mit Eiswasser gewaschen und gut abgepresst und danach mit 0,02 l Aceton verrieben und abgenutscht. Dieser Vorgang wurde noch zweimal wiederholt. Der dunkelbraune feinkristalline Rückstand wurde noch zweimal mit Wasser verrieben, abgenutscht und auf der Nutsche mit Wasser nachgewaschen. Die gelbraunen Kristalle wurden bei 80 °C 20 Stunden im Wasserstrahlvakuum getrocknet. Man erhielt 18 g o-(6-Hydroxy-3-oxo-3H-thioxanthen-9-yl)-benzolsulfonsäure, Schmelzpunkt

über 300 °C (Zers.)., Absorptionsmaximum des Dinatriumsalzes in Methanol : $\lambda_{max}$ = 524 nm (ε = 24 430).

Beispiel 7

5,0 g m,m'-Selenodiphenol und 5,85 g Phthalsäureanhydrid wurden auf 80 °C erwärmt. Die Schmelze versetzte man unter Rühren mit 5 ml Trifluormethansulfonsäure, wobei die Temperatur im Reaktionsgefäss auf 120 °C anstieg und das Reaktionsgemisch schwarz wurde. Das Reaktionsgemisch wurde 1 Stunde in einem Oelbad auf ca. 90 °C (Innentemperatur) gehalten, nach dem Abkühlen 2 Stunden bei 60 °C mit 100 ml 2N Natronlauge gerührt, filtriert und der Rückstand mit 150 ml Wasser gewaschen. In das Filtrat tropfte man innert 40 Minuten unter Rühren 150 ml 2N Schwefelsäure und liess die Lösung 16 Stunden unter Lichtausschluss bei Raumtemperatur stehen. Die Suspension wurde auf ca. 250 ml eingeengt, abgenutscht und der Rückstand auf der Nutsche mit 100 ml Wasser gewaschen, mit 250 ml 3,5 % Natriumhydrogencarbonatlösung vom Filter heruntergelöst, 30 Minuten bei Raumtemepratur gerührt und über ein Filterhilfsmittel filtriert. Das Filtrat wurde auf ca. 300 ml eingeengt und während 1,25 Stunden unter Rühren mit 115 ml 2N Schwefelsäure versetzt. Danach wurde die Lösung auf ca. 200 ml eingeengt, abgenutscht und der Rückstand erst mit Wasser und schliesslich mit Hexan gut gewaschen. Der Rückstand wurde dann in wenig Methanol gelöst und die Lösung auf präparative Schichtchromatographie (= PSC)-Platten aufgetragen. Nach Elution mit Dichlormethan/Essigsäureäthylester 1 : 1 wurde die an der Farbe erkennbare Zone von den Platten abgekratzt, in kaltem Methanol aufgeschlämmt, filtriert, das Filtrat zur Trockne eingedampft und die Prozedur mit Aceton wiederholt. Man erhielt 2,33 g o-(6-Hydroxy-3-oxo-3H-selenoxanthen-9-yl)-benzoesäure. 1 Aceton in roten Kristallen vom Schmelzpunkt 150 °C (Zers.). Acetonfreies Dinatriumsalz in Methanol : $\lambda_{max}$ = 526 nm (ε = 18 510).

Beispiel 8

3,59 g p-Toluolsulfonsäure-monohydrat wurden wie in Beispiel 6 bei 140 °C und 0,1 Torr entwässert. Ein Gemisch von 5 g m,m'-Selendiphenol und 3,47 g 2-Sulfobenzoesäureanhydrid wurde in die heisse Schmelze der p-Toluolsulfonsäure eingetragen. Das Reaktionsgemisch wurde 2,5 Stunden bei 140 °C (Innentemperatur) und 0,01-0,05 Torr gehalten. Nach dem Abkühlen löste man die halbfeste Masse bei 50 °C in 150 ml Ammoniak 25 %, engte auf ca. 75 ml ein, versetzte unter Rühren mit 250 ml Wasser und liess die dunkelrote Lösung 16 Stunden unter Lichtausschluss bei Raumtemperatur stehen. Nach Zugabe von ca. 1 g Aktivkohle wurde filtriert und mit 200 ml Wasser nachgewaschen. Danach wurde die Lösung bei 60 °C auf ca. 400 ml eingeengt und im Verlauf von 1,8 Stunden unter Rühren tropfenweise mit einer Mischung aus 7,5 ml Schwefelsäure 95-97 % in 30 ml Wasser versetzt. Das Produkt fiel als sehr feiner rotbrauner Niederschlag aus, welcher nach 0,5 Stunden abgenutscht und auf der Nutsche dreimal mit je 40 ml Diäthyläther gewaschen wurde. Das Kristallisat wurde in 150 ml Aethanol aufgenommen und das Lösungsmittel bei 50 °C i. V. zur Trockne abgedampft. Man erhielt rohe o-(6-Hydroxy-3-oxo-3H-selenoxanthen-9-yl)-benzolsulfonsäure.

3,13 g Rohprodukt wurden in 200 ml Ammoniak 25 % gelöst, i. V. bei 50 °C auf 100 ml eingeengt und mit 500 ml Wasser versetzt. Nach Filtration der Lösung wurde auf ca. 300 ml eingeengt. Danach wurden 7,5 ml Schwefelsäure 95-97 % in 30 ml Wasser im Verlauf von 0,5 Stunden zugesetzt, der Niederschlag nach 16 Stunden über ein Papierfilter abgenutscht, mit 100 ml Wasser sowie mit 100 ml Diäthyläther gewaschen, in 150 ml Aethanol aufgenommen und die Aufschlämmung bei 60 °C i. V. total eingedampft. Man erhielt 2,70 g Produkt, welches nach DC noch immer gefärbte Verunreinigungen enthielt. Dieses Produkt wurde in 200 ml Ammoniak 25 % gelöst. Die Lösung wurde zur Trockne eingedampft, das Salz in 6 ml Wasser gelöst und unter Rühren mit 60 ml 2-Propanol und anschliessend mit 80 ml Aceton versetzt. Zur Vervollständigung der Kristallisation wurden 50 ml Lösungsmittel abgedampft und die eingeengte Lösung 16 Stunden bei Raumtemperatur stehen gelassen.

Dann nutschte man ab und wusch mit Aceton gut nach. Das Salz wurde in 100 ml Wasser gelöst und das Produkt mit einer Mischung aus 7,5 ml Schwefelsäure 95-97 % und 30 ml Wasser innerhalb von 1,2 Stunden gefällt. Nach 1-stündigem Stehen wurde abgenutscht, mit Diäthyläther gut gewaschen und das Kristallisat 20 Stunden bei 50 °C im Wasserstrahlvakuum getrocknet. Man erhielt 1,40 g dünnschichtchromatographisch reine o-(6-Hydroxy-3-oxo-3H-selenoxanthen-9-yl) benzolsulfonsäure. Dinatriumsalz in Methanol : $\lambda_{max}$ = 533 nm (ε = 35 540).

**Patentansprüche**

1. Verfahren zur Herstellung von Prävitamin D, das im wesentlich frei von Tachysterin ist, dadurch gekennzeichnet, dass man ein Gemisch von Prävitamin D und Tachysterin in Gegenwart einer Verbindung der Formel

(Siehe Figur Seite 6 f.)

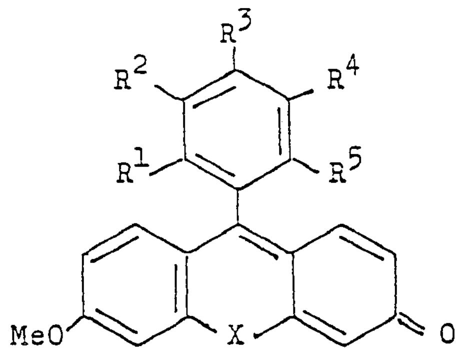

(I)

worin $R^1$-$R^4$ Wasserstoff oder Chlor, $R^5$ eine Gruppe —COOMe oder —$SO_3$Me, Me ein Alkalimetallkation und X Sauerstoff, Schwefel oder Selen bedeuten, mit Licht einer Wellenlänge von 400 nm bis 600 nm bestrahlt.

2. Verfahren nach Anspruch 1, dadurch gekennzeichnet, dass man ein durch UV-Bestrahlung von Provitamin D erhaltenes Gemisch, das Prävitamin D und Tachysterin enthält, bestrahlt.

3. Verfahren nach den Ansprüchen 1 oder 2, dadurch gekennzeichnet, dass die Lichtquelle eine mit einem die Wellenlängen unterhalb 365 nm eliminierenden Filter versehene Quecksilberhochdrucklampe ist.

4. Verfahren nach Anspruch 3, dadurch gekennzeichnet, dass das Filter eine Flüssigkeit ist.

5. Verfahren nach den Ansprüchen 1-4, dadurch gekennzeichnet, dass man eine Verbindung der Formel I verwendet, in der $R^1$-$R^4$ Wasserstoff, $R^5$ eine Gruppe —COONa oder —$SO_3$Na, und X Schwefel oder Selen ist.

6. Verbindungen der Formel I, in denen Me und $R^1$-$R^5$ die in Anspruch 1 angegebene Bedeutung haben, X Schwefel oder Selen ist und $R^5$ eine Gruppe —$SO_3$Me ist, wenn X Schwefel darstellt.

7. o-(6-Hydroxy-3-oxo-3H-thioxanthen-9-yl)-benzolsulfonsäure und deren Dinatriumsalz.

8. o-(6-Hydroxy-3-oxo-3H-selenoxanthen-9-yl)-benzoesäure und deren Dinatirumsalz.

9. o-(6-Hydroxy-3-oxo-3H-selenoxanthen-9-yl)-benzol-sulfonsäure und deren Dinatriumsalz.

10. Verwendung von Verbindungen gemäss den Ansprüchen 6-9 als Photosensibilisatoren.

**Claims**

1. A process for the manufacture of previtamin D which is sbustantially free from tachysterol, characterized by irradiating a mixture of previtamin D and tachysterol with light of a wavelength of 400 nm to 60 nm in the presence of a compound of the formula

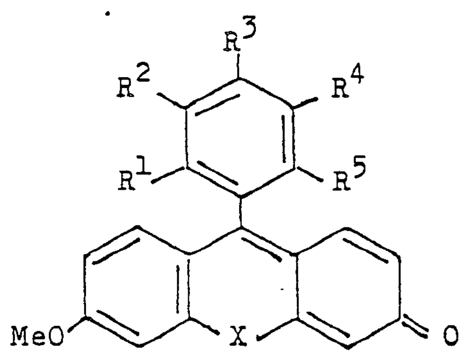

(I)

wherein $R^1$-$R^4$ signify hydrogen or chlorine, $R^5$ signifies a group —COOMe or —$SO_3$Me, Me signifies an alkali metal càtion and X signifies oxygen, sulphur or selenium.

2. A process according to claim 1, characterized in that a mixture which contains previtamin D and tachysterol and which is obtained by the UV-irradiation of provitamin A is irradiated.

3. A process according to claims 1 or 2, characterized in that the light source is a mercury highpressure lamp provided with a filter which eliminates the wavelengths below 365 nm.

4. A process according to claim 3, characterized in that the filter is a liquid filter.

5. A process according to claims 1-4, characterized in that a compound of formula I in which $R^1$-$R^4$ are hydrogen, $R^5$ is a group —COONa or —$SO_3$Na and X is sulphur or selenium is used.

6. Compounds of formula I in which Me and $R^1$-$R^5$ have the significance given in claim 1, X is sulphur or selenium and $R^5$ is a group —$SO_3$Me when X represents sulphur.

7. o-(6-Hydroxy-3-oxo-3H-thioxanthen-9-yl)-benzene-sulphonic acid and its disodium salt.

8. o-(6-Hydroxy-3-oxo-3H-selenoxanthen-9-yl)-benzoic acid and its disodium salt.

9. o-(6-Hydroxy-3-oxo-3H-selenoxanthen-9-yl)-benzene-sulphonic acid and its disodium salt.

10. The use of compounds in accordance with claims 6-9 as photosensitizers.

## Revendications

1. Procédé de préparation de la pré-vitamine D essentiellement exempte de tachystérol, caractérisé en ce que l'on expose un mélange de pré-vitamine D et de tachystérol à la lumière d'une longueur d'onde de 400 nm à 600 nm en présence d'un composé de formule :

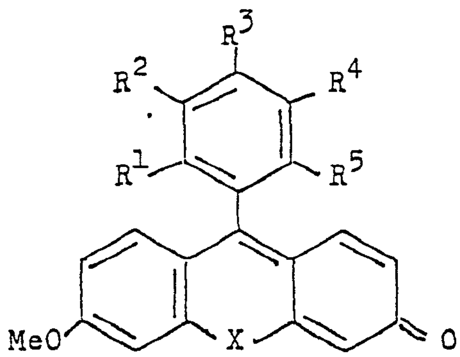

(I)

dans laquelle les symboles $R^1$ à $R^4$ représentent l'hydrogène ou le chlore, $R^5$ représente un groupe —COOMe ou —$SO_3$Me, Me représente un cation de métal alcalin et X représente l'oxygène, le soufre ou le sélénium.

2. Procédé selon la revendication 1, caractérisé en ce que l'on expose à la lumière un mélange, contenant de la pré-vitamine D et du tachystérol, obtenu par exposition de la pro-vitamine D à la lumière ultraviolette.

3. Procédé selon les revendications 1 ou 2, caractérisé en ce que la source de lumière est une lampe à mercure à haute pression équipée d'un filtre supprimant les longueurs d'ondes inférieures à 365 nm.

4. Procédé selon la revendication 3, caractérisé en ce que le filtre consiste en un liquide.

5. Procédé selon les revendications 1 à 4, caractérisé en ce que l'on utilise un composé de formule I dans laquelle les symboles $R^1$ à $R^4$ représentent l'hydrogène, $R^5$ représente un groupe —COONa ou —$SO_3$Na, et X représente le soufre ou le sélénium.

6. Composés de formule I dans laquelle Me et $R^1$ à $R^5$ ont les significations indiquées dans la revendication 1, X représente le soufre ou le sélénium et $R^5$ représente un groupe —$SO_3$Me lorsque X représente le soufre.

7. L'acide o-(6-hydroxy-3-oxo-3H-sélénoxanthène-9-yl)-benzènesulfonique et son sel disodique.

8. L'acide o-(6-hydroxy-3-oxo-3H-sélénoxanthène-9-yl)-benzoïque et son sel disodique.

9. L'acide o-(6-hydroxy-3-oxo-3H-sélénoxanthène-9-yl)-benzènesulfonique et son sel disodique.

10. L'utilisation des composés selon les revendications 6 à 9 en tant que photosensibilisants.